(19) Europäisches Patentamt
European Patent Office
Office européen des brevets

(11) EP 0 643 698 B1

(12) FASCICULE DE BREVET EUROPEEN

(45) Date de publication et mention de la délivrance du brevet: **05.03.1997 Bulletin 1997/10**

(21) Numéro de dépôt: **93902331.3**

(22) Date de dépôt: **15.12.1992**

(51) Int Cl.[6]: **C07D 215/18**, C07D 215/28, G01J 3/00, G01N 21/00

(86) Numéro de dépôt international: **PCT/FR92/01184**

(87) Numéro de publication internationale: **WO 93/13071 (08.07.1993 Gazette 1993/16)**

# (54) PROCEDES SPECTROPHOTOMETRIQUES DE DOSAGE DES MERCAPTANS TOTAUX, DU GLUTATHION REDUIT (GSH) ET DES MERCAPTANS AUTRES QUE LE GSH DANS UN MILIEU AQUEUX, REACTIFS ET NECESSAIRES POUR LEUR MISE EN OEUVRE

VERFAHREN ZUR SPEKTRALPHOTOMETRISCHEN BESTIMMUNG VON GESAMTEN MERKAPTANEN, VON REDUZIERTEM GLUTATHION (GSH) UND VON MERKAPTANEN, UNGLEICH GSH, IN EINEM WAESSRIGEN MEDIUM, REAGENZIEN UND KITS FUER DEREN DURCHFUEHRUNG

SPECTROPHOTOMETRIC METHODS FOR ASSAYING TOTAL MERCAPTANS, REDUCED GLUTATHIONE (GSH) AND MERCAPTANS OTHER THAN GSH IN AN AQUEOUS MEDIUM, REAGENTS AND KITS FOR THEIR IMPLEMENTATION

(84) Etats contractants désignés:
**DE GB IT**

(30) Priorité: **20.12.1991 FR 9115868**

(43) Date de publication de la demande:
**22.03.1995 Bulletin 1995/12**

(73) Titulaire: **OXIS INTERNATIONAL S.A.
94385 Bonneuil sur Marne (FR)**

(72) Inventeurs:
- **YADAN, Jean-Claude, Yomtob
F-75011 Paris (FR)**
- **ANTOINE, Marc, Gabriel
F-77420 Champs-sur-Marne (FR)**
- **CHAUDIERE, Jean, Raymond
F-94100 Saint-Maur-des-Fosses (FR)**

(74) Mandataire: **Portal, Gérard et al
Cabinet Beau de Loménie
158, rue de l'Université
75340 Paris Cédex 07 (FR)**

(56) Documents cités:
- **JOURNAL OF CHROMATOGRAPHY, vol. 553, no. 1-2, 1991, AMSTERDAM, NL; pages 433-439, B. LIN LING ET AL.: 'Micro liquid chromatography with fluorescence detection of thiols and disulphides'.**
- **CHEMICAL ABSTRACTS, vol. 69, no. 4, 22 Juillet 1968, Columbus, Ohio, US; abstract no. 12969k, A.W. DAVIDSON, 'Chlorinated hydrocarbons in drugs: chloral hydrate.' page 1239.**
- **CHEMICAL ABSTRACTS, vol. 73, no. 8, 24 Août 1970, Columbus, Ohio, US; abstract no. 38604v, L.P. KRAVCHENYUK ET AL.: 'Polarography of physiologically active ammonium-substituted thiocyanate salts. I. Benzylpyridinium and benzylquinolinium thiocyanates.' page 228.**
- **CHEMICAL ABSTRACTS. REGISTRY HANDBOOK - NUMBER SECTION. COLUMBUS US '1965-1971' *CAS RN 316-74-5***
- **CHEMICAL ABSTRACTS. REGISTRY HANDBOOK - NUMBER SECTION. 1984, COLUMBUS, US; *CAS RN 93688-89-2***
- **CHEMICAL ABSTRACTS. REGISTRY HANDBOOK - NUMBER SECTION. 1985, COLUMBUS, US; *CAS RN 97556-25-7***
- **CHEMICAL ABSTRACTS. REGISTRY HANDBOOK - NUMBER SECTION. 1988, COLUMBUS, US; *CAS RN 114306-20-6, 114382-38-6***

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne le dosage des mercaptans et du glutathion réduit, notamment dans les milieux biologiques.

Elle a plus particulièrement pour objet un procédé spectrophotométrique de dosage des mercaptans totaux, du glutathion réduit (en abrégé GSH) et, par différence, des mercaptans autres que le GSH, dans un milieu aqueux, notamment biologique, des réactifs et des nécessaires ou kits pour la mise en oeuvre de ce procédé.

Le glutathion, composé ubiquitaire, est une molécule-clef sur le plan physiologique en raison de ses nombreuses fonctions biochimiques (référence 1). En conséquence, les variations de sa concentration, en particulier une diminution de son taux intracellulaire normal (1-10 mM) , peuvent entraîner des désordres importants. Il est donc nécessaire de pouvoir disposer d'une méthode de dosage du GSH qui soit fiable, simple, sensible et spécifique de ce paramètre biochimique.

L'existence de nombreuses méthodes de dosage du GSH suggère que ces dernières ne sont pas satisfaisantes (référence 2).

Les techniques de mesure connues des mercaptans, en général, et du GSH en particulier, exploitent l'une ou l'autre des deux principales propriétés chimiques des mercaptans : pouvoir réducteur et pouvoir nucléophile, d'où leur classification en :

- Méthodes utilisant le pouvoir réducteur des mercaptans :
  Ces méthodes ne sont pas spécifiques du GSH, elles nécessitent donc une étape de purification chromatographique pour doser le GSH et, dans certains cas, la présence d'une enzyme de couplage comme par exemple la glutathion-réductase (référence 3) ; et
- Méthodes utilisant le pouvoir nucléophile des mercaptans :
  Ces méthodes reposent sur la substitution de dérivés halogénés (de type dinitrohalogénobenzène ou monohalogénobimane, par exemple) avec ou sans glutathiontransférase. Ces méthodes, également, ne sont pas spécifiques du GSH en absence d'enzyme et nécessitent une étape de séparation chromatographique pour doser le GSH.

Selon les cas, la détection peut être de type spectrophotométrique, spectrofluorimétrique (référence 4), ou électrochimique (référence 5).

Dans l'exposé qui suit, on se limitera aux méthodes utilisant la spectrophotométrie d'absorption UV/visible ou la spectrofluorimétrie comme technique de détection.

Les méthodes utilisant le pouvoir réducteur des mercaptans sont basées sur un échange thiol-disulfure, selon le mécanisme réactionnel suivant (dans le cas du glutathion) :

$$2\ GS^- + RSSR \rightarrow GSSG + 2\ RS^-$$

La plus connue utilise le réactif d'ELLMAN (références 6 et 7). Cette méthode basée sur la formation d'un chromophore à 412 nm est peu complexe, mais l'instabilité du réactif d'ELLMAN vis-à-vis de la lumière et du pH (hydrolyse), et l'autoxydation du chromophore libéré nécessitent la réalisation d'une mesure de référence qui alourdit le test. Tout ceci en fait un réactif non utilisable en Biologie Clinique sur de grandes séries d'échantillons.

L'analogue sélénié du réactif d'ELLMAN a été synthétisé et utilisé pour le dosage de mercaptans (référence 8). Il est plus résistant que le réactif d'ELLMAN vis-à-vis de l'hydrolyse alcaline mais présente, par ailleurs, les mêmes inconvénients que ce dernier.

Enfin, il convient de mentionner l'utilisation de disulfures pyridiniques (référence 9).

D'une façon générale, les réactifs d'échange thiol-disulfure ne peuvent pas être utilisés pour un dosage spécifique du GSH.

Les méthodes basées sur le pouvoir nucléophile des mercaptans exploitent leur capacité d'addition sur des réactifs électrophiles, à savoir :

1° <u>Les réactifs halogénoaromatiques activés</u> :

Ces réactifs du type 1-chloro-2,4-dinitrobenzène (CDNB) ou 2,4-dinitro-1-fluoro-benzène (DNFB) ne sont pas spécifiques des groupements thiols à pH alcalin. Ils nécessitent la présence d'une enzyme telle que la GSH-transférase, dont ils servent par ailleurs à mesurer l'activité. La faible sensibilité de ces réactifs est l'un des inconvénients majeurs de ces méthodes. De plus, leur réactivité non enzymatique n'est pas nulle, ce qui exclut un dosage totalement spécifique du GSH. Pour pallier cet inconvénient, de nombreuses méthodes sont basées sur la séparation chromatographique des produits réactionnels comme, par exemple, la méthode de chromatographie liquide haute performance (HPLC)

de REED (références 10 et 11) qui couple une formation de dérivés, ou dérivatisation, des amines (à l'aide du DNFB : réactif de Sanger) à une S-carboxyméthylation, mais qui est longue et lourde à mettre en oeuvre.

2° Les monohalogénobimanes (référence 12) :

Ces composés peuvent réagir avec des groupements nucléophiles autres que les groupements thiols, tels que par exemple les amines. Ils ne sont pas, non plus, spécifiques du GSH en présence d'autres mercaptans : il est donc, ici encore, nécessaire d'intégrer dans le protocole une étape de séparation chromatographique. Ce procédé permet néanmoins d'obtenir un profil exhaustif des mercaptans présents. Toutefois, la photosensibilité des réactifs et des produits de réaction implique des précautions d'emploi qui alourdissent la méthode de dosage.

Par ailleurs, la cinétique lente du monochlorobimane, à pH inférieur ou égal à 8, permet d'exploiter la spécificité biochimique des glutathion-S-transférases au voisinage de la neutralité. Ce réactif pénètre lentement les cellules intactes, à l'intérieur desquelles il réagit avec l'enzyme et son substrat le GSH, ce qui permet un dosage en cytométrie de flux (référence 13). Le dérivé monobromotriméthylammoniobimane (référence 14) présente, quant à lui, l'avantage d'une plus grande solubilité dans l'eau et permet une détection conductimétrique, mais cette dernière est difficile à standardiser.

Quels que soient leurs avantages particuliers, d'une façon générale, la plupart de ces méthodes requièrent une séparation chromatographique sophistiquée, et leur mise en oeuvre est trop longue pour permettre l'analyse rapide d'une grande série d'échantillons.

3° Les dérivés de l'acide iodoacétique :

Ces composés ont une spécificité inférieure à celle des réactifs cités ci-dessus, leur sensibilité est plutôt moyenne, voire médiocre dans certains cas. Ils sont généralement instables à la lumière et nécessitent une séparation chromatographique en raison de leurs caractéristiques d'absorption similaires à celles du produit d'addition.

4° Les dérivés du maléimide :

La spécificité de ces composés n'est pas meilleure que celle des réactifs précédents. Leur sensibilité n'est bonne que dans le cas où le produit d'addition est fluorescent.

L'un des inconvénients majeurs de cette dernière méthode est que l'on suit une diminution d'absorption préexistante, ce qui est moins sûr et plus délicat que de suivre l'augmentation d'une nouvelle absorption.

5° Les réactifs possédant une double liaison électrophile activée (accepteurs de Michaël) :

Ces réactifs incluent :

- les énones (référence 15) qui réagissent très lentement avec les composés nucléophiles azotés ou oxygénés, mais plus rapidement avec les composés nucléophiles soufrés; leur spécificité n'est jamais totale vis-à-vis des groupements thiols; leur sensibilité, quant à elle, est variable selon le réactif considéré; elles présentent généralement les mêmes inconvénients majeurs que les réactifs précédents;
- les vinylpyridines qui sont des réactifs lents, peu solubles en milieu aqueux, utilisés pour bloquer le groupement SH des mercaptans, en général, et du glutathion (référence 16), en particulier, mais non pour le doser.

Il convient enfin de citer deux types de réactifs fluorescents qui sont inadaptés à un dosage de routine pour des raisons de complexité méthodologique et de spécificité limitée vis-à-vis des groupements thiols en général et inexistante vis-à-vis du glutathion, ce sont :

- l'aldéhyde orthophtalique qui requiert la présence d'un groupement thiol et d'une amine primaire pour fournir un dérivé isoindolique fluorescent (références 17 et 18) ; et
- les dérivés du benzoxa-1,3-diazole, en particulier le 7-chloro-2-benzoxa-1,3-diazole-4-sulfonate (SBD-Cl) et le 7-fluoro-2-benzoxa-1,3-diazole-4-sulfonate (SBD-F), qui requièrent un temps de réaction important et un pH alcalin de 9,5 (références 19 et 20).

L'invention a pour but de remédier aux inconvénients des méthodes connues de dosage des mercaptans en général et du GSH en particulier en fournissant :

- des réactifs colorimétriques spécifiques des mercaptans dans des échantillons, notamment biologiques, bruts et

susceptibles de permettre un dosage spécifique du GSH en présence de mercaptans contaminants, et rapides, c'est-à-dire dont le temps de réaction totale avec les mercaptans du milieu soit inférieur à 15 minutes dans les conditions opératoires;
- un procédé d'utilisation de tels réactifs permettant de doser, sur une seule prise d'échantillon, les mercaptans totaux dans une première étape et le GSH réduit dans une seconde étape, ce qui aboutirait à la détermination de trois paramètres physiologiques :

  * la quantité de mercaptans totaux
  * la quantité de GSH
  * la quantité de mercaptans distincts du GSH, par différence.

L'invention a, en particulier, pour but de mettre au point un procédé simple et rapide de mesure au GSH réduit, adapté à un dosage de routine sur de grandes séries d'échantillons, ce qui implique :

* la formation d'un chromophore absorbant le plus souvent dans le visible, dont la mesure de l'absorbance ne nécessite pas de spectrophotomètre spécifique ;
* un dosage en point final et non en cinétique ;
* un temps d'incubation court : par exemple inférieur à 30 minutes ;
* des conditions opératoires permettant d'annuler le risque d'autoxydation des groupements thiols pendant la durée du dosage.

Ces buts sont atteints grâce à l'invention qui, selon l'un de ses modes de réalisation, fournit un procédé de dosage des mercaptans dans un échantillon aqueux, notamment biologique, caractérisé en ce qu'il comprend essentiellement la formation d'un produit de substitution chromophorique entre tous les mercaptans présents dans l'échantillon et un réactif choisi parmi les composés de type quinoléinium répondant à la formule générale I :

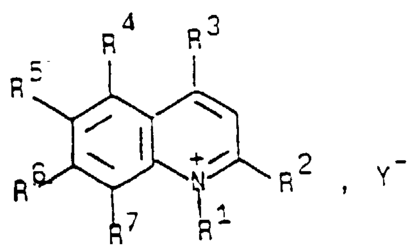

(I)

dans laquelle :

$R^1$ = alkyle (avec 1 à 6 atomes de carbone), benzyle, p-nitrobenzyle, phényle ou o,p-dinitrophényle ;
$R^2$ = hydrogène ou méthyle ;
$R^3$ = hydrogène, F, Cl, Br, I ou SCN ;
$R^4$ = hydrogène, F, Cl, $NO_2$ ou $CF_3$ ;
$R^5$ = hydrogène, $NO_2$ ou Cl ;
$R^6$ = hydrogène, F, Cl, $CF_3$ ou $NO_2$ ;
$R^7$ = hydrogène, OH, benzyloxy ou $NO_2$;

étant entendu que $R^3$, $R^4$, $R^5$ et $R^6$ ne peuvent représenter simultanément l'hydrogène, et

Y = fluorure, chlorure, bromure, iodure, méthylsufate, fluorosulfonate, phosphate, tétrafluoroborate ou tosylate.

Les composés de formule générale I ci-dessus peuvent être préparés, à partir de composés commerciaux, par analogie à des procédés connus et notamment comme suit.

Les dérivés de la série fluoroquinoléinium sont obtenus par réaction de Schiemann sur les sels de diazonium correspondants, préparés eux-mêmes à partir de composés commerciaux.

Une procédure générale de la dernière étape de synthèse des composés de formule générale I est réalisée comme suit.

Au dérivé quinoléinique (1 mmole) dissous dans 10 volumes d'un solvant organique, comme par exemple l'acétonitrile, est ajouté à 10°C l'agent alkylant soit pur, comme par exemple l'iodure de méthyle, soit en solution dans un solvant organique, comme par exemple le tétrafluoroborate de triméthyloxonium (2 mmoles) dans 1 volume d'acétonitrile. Le milieu réactionnel est agité pendant 30 minutes à 2 heures. Le solvant est évaporé sous pression réduite,

le résidu est repris par 1 volume d'éther terbutylméthylique, puis essoré, lavé et séché sous vide.

Lorsque cela s'avère nécessaire, les produits sont purifiés par chromatographie liquide haute performance (HPLC) préparative sur colonne de silice (C8, 5µm), en éluant par un mélange eau/acétonitrile. Une lyophilisation finale permet d'aboutir aux produits désirés avec des rendements non optimisés compris entre 20 et 89 %.

Lorsque cela s'avère approprié, l'alkylation est réalisée dans l'iodure de méthyle au reflux, ou à l'aide de sulfate de méthyle dans l'acétone au reflux ou de fluorosulfonate de méthyle dans un solvant organique, tel que par exemple le glyme, selon le produit final désiré.

Selon un autre mode de réalisation, l'invention fournit un procédé de dosage du glutathion réduit (GSH) dans un échantillon aqueux, notamment biologique, caractérisé en ce qu'il comprend essentiellement les étapes consistant à :

1. former un produit de substitution chromophorique entre tous les mercaptans présents dans l'échantillon et un réactif choisi parmi les composés de formule générale I définie ci-dessus ; et
2. amener le pH du milieu réactionnel à une valeur comprise entre 12,8 et 13,8, pour former spécifiquement une thione chromophorique à partir du produit de substitution chromophorique formé à l'étape 1. entre le GSH et ledit réactif.

Le principe du dosage du glutathion réduit selon l'invention repose donc sur une stratégie comprenant deux étapes chimiques consécutives :

- tout d'abord, formation d'un produit de substitution, appelé ci-après "adduit", entre tous les mercaptans (RSH) présents dans l'échantillon, dont notamment le glutathion réduit (GSH), et l'un des réactifs définis ci-dessus, appelé "réactif BXT", selon la réaction :

$$\text{RSH + réactif BXT} \rightarrow \text{RS-BXT (chromophore 1)}$$

- puis, après alcalinisation du milieu, à pH compris entre 12,8 et 13,8, selon le réactif BXT utilisé, formation, par réaction de β-élimination, d'une thione chromophorique, spécifique de l'adduit GS-BXT, formé à partir du GSH et du réactif BXT, selon la réaction :

$$\text{GS-BXT} \xrightarrow{\text{pH alcalin}} \text{thione (chromophore 2).}$$

Cette méthode permet de déterminer, au moyen de mesures spectrophotométriques :

* dans un premier temps, la quantité de mercaptans totaux (RSH) présents dans l'échantillon ;
* dans un deuxième temps, la quantité de glutathion réduit (GSH) dans ce même échantillon ; et
* par différence, la quantité de mercaptans distincts du glutathion réduit.

Cette méthode permet donc d'avoir accès à trois paramètres à l'aide d'une seule prise d'échantillon.

L'originalité du procédé de dosage selon l'invention est basée sur une stratégie comprenant une première étape de formation d'un adduit chromophorique entre le réactif BXT et tous les mercaptans présents dans l'échantillon et une seconde étape, après alcalinisation, de formation d'une thione chromophorique, spécifiquement avec l'adduit GSH/réactif BXT.

L'utilisation des réactifs et du procédé décrits permet donc de mesurer, sur une seule prise d'échantillon, la quantité globale de mercaptans, en général, et celle du GSH, en particulier, en milieu aqueux, à l'aide d'une double mesure colorimétrique. La simplicité de mise en oeuvre de cette méthode originale est très supérieure à celle des méthodes spectrophotométriques préalablement décrites. La méthode de dosage colorimétrique du GSH qui est décrite ici, est la première qui soit totalement spécifique, en l'absence de tout réactif enzymatique.

Cette nouvelle méthode de dosage constitue donc un nouvel outil pour les recherches biologiques en général ainsi qu'en Chimie Clinique, en particulier pour l'élaboration de kits de dosage ou de diagnostic, les physiopathologies potentielles étant par exemple la cataracte, le diabète, la polyarthrite rhumatoïde, l'ischémie.

Les échantillons visés sont notamment les érythrocytes, le plasma, les plaquettes sanguines, et d'une façon générale tout échantillon, notamment biologique, contenant des mercaptans et en particulier du GSH.

Selon un mode avantageux de mise en oeuvre, l'invention a pour objet un procédé de dosage des mercaptans totaux dans un échantillon aqueux, notamment biologique, caractérisé en ce qu'il comprend essentiellement les étapes consistant à :

1. ajouter à l'échantillon à doser, tamponné dans la gamme de pH 7,0 à 7,5, un aliquot d'une solution-mère, dans l'eau ou dans un solvant miscible à l'eau, d'un réactif BXT de formule générale I en excès,
2. homogénéiser le milieu réactionnel ainsi obtenu,
3. laisser incuber pendant au moins 5 minutes,
4. mesurer, à l'aide d'un spectrophotomètre, l'absorbance du ou des produit(s) de substitution ou adduit(s) formé(s) entre le réactif BXT et le ou les mercaptan(s) présent(s) dans l'échantillon et
5. déterminer la concentration en moles/litre des mercaptans totaux présents dans l'échantillon, selon la formule :

$$[\text{mercaptans totaux}] = (A/\varepsilon m \times d) \times D$$

dans laquelle :

- A représente l'absorbance mesurée,
- εm représente le coefficient d'extinction molaire spécifique du réactif BXT utilisé, exprimé en $l.mole^{-1}.cm^{-1}$,
- d représente la longueur de la cuve, exprimée en cm, et
- D représente le coefficient de dilution.

Selon un autre mode avantageux de mise en oeuvre, l'invention a pour objet un procédé de dosage du glutathion réduit, GSH, dans un échantillon aqueux, notamment biologique, caractérisé en ce qu'il comprend essentiellement les étapes consistant à :

1. ajouter à l'échantillon à doser, tamponné dans la gamme de pH 7,0 à 7,5, un aliquot d'une solution-mère, dans l'eau ou dans un solvant miscible à l'eau, d'un réactif BXT de formule générale I en excès,
2. homogénéiser le milieu réactionnel ainsi obtenu,
3. laisser incuber pendant au moins 5 minutes,
4. ajuster le pH du mélange à une valeur comprise entre 12,8 et 13,8, selon le réactif BXT choisi,
5. laisser incuber pendant au moins 15 minutes,
6. mesurer, à l'aide d'un spectrophotomètre, l'absorbance de la thione chromophorique produite par β-élimination, spécifiquement avec l'adduit GSH-réactif BXT et
7. déterminer la concentration en moles/litre du GSH dans l'échantillon, selon la formule :

$$[GSH] = (A'/\varepsilon m \times d) \times D$$

dans laquelle :

- A' représente l'absorbance mesurée,
- εm représente le coefficient d'extinction molaire spécifique du réactif BXT utilisé, exprimé en $l.mole^{-1}.cm^{-1}$,
- d représente la longueur de la cuve, exprimée en cm, et
- D représente le coefficient de dilution.

Selon encore un autre mode de réalisation avantageux de l'invention, on détermine la concentration des mercaptans distincts du glutathion réduit présents dans l'échantillon, au moyen de la différence des concentrations mesurées selon les deux procédés définis ci-dessus, c'est-à-dire en utilisant la formule :

$$[\text{mercaptans distincts du GSH}] = [\text{mercaptans totaux}] - [GSH].$$

Le dosage des mercaptans totaux, du glutathion réduit et des mercaptans autres que le GSH dans un échantillon aqueux, notamment biologique, à l'aide des nouveaux réactifs de formule générale I, repose donc sur la mesure spectrophotométrique de la formation des nouveaux chromophores 1 et 2, produits respectivement au cours de chacune des deux réactions constituant cette méthode originale.

Des méthodes générales qui peuvent être utilisées pour la mise en oeuvre de ces déterminations sont expliquées plus en détail ci-après.

<u>Dosage des mercaptans totaux</u> :

Le milieu réactionnel est constitué par un tampon de pH = 7,0 à 7,5, comme, par exemple, un tampon phosphate 50 mM, contenant de l'acide diéthylènetriaminepentacétique (DTPA) 1 mM. Selon la nature du tampon considéré, une addition de soude ou d'acide chlorhydrique, à température ambiante, permet d'ajuster le pH à la valeur désirée. La température de travail choisie, par exemple 25°C, doit être maintenue constante (±3°C) durant les temps d'incubations et de mesures. Les solutions tampons et de réactifs doivent être préparées et conservées entre 0 et 4°C. Dans ces conditions, ces solutions sont stables pendant 3 mois. Le rapport concentration en réactif BXT/concentration en mercaptans estimée doit être supérieur ou égal à 10 et ne dépasse en général pas 1000, pour garantir des temps réactionnels courts.

La réaction est déclenchée par l'ajout, par exemple dans une cuve pour spectrophotomètre contenant l'échantillon à doser, d'un aliquot d'une solution-mère du réactif BXT dans l'eau ou dans un solvant organique miscible à l'eau, tel que par exemple l'éthanol, l'acétonitrile, le diméthylformamide ou le diméthylsulfoxyde. Le volume réactionnel final, par exemple 1 ml, ne doit pas varier d'une mesure à l'autre.

Après homogénéisation de la solution et incubation pendant 5 à 10 minutes à la température de travail choisie, l'absorbance à la longueur d'onde de l'adduit ou des adduits est mesurée: par exemple à 356 nm pour l'adduit entre le composé BXT03015 selon l'invention, décrit dans l'exemple 1 de la partie expérimentale qui suit, et le GSH.

Le tableau I qui suit contient, à titre d'exemples, les données spectrophotométriques obtenues avec le réactif BXT03015 et un certain nombre de mercaptans physiologiques ou susceptibles d'être utilisés en biochimie ou en Chimie Clinique.

TABLEAU I

| Données spectrales des chromophores obtenus par réaction entre le réactif BXT03015 et quelques mercaptans, avant et après alcalinisation | | |
|---|---|---|
| | AVANT | APRES |
| | alcalinisation | |
| Mercaptans | Adduit de BXT03015 | |
| | λmax(nm), | λmax(nm), |
| | εm($l.mole^{-1}.cm^{-1}$) | εm($l.mole^{-1}.cm^{-1}$) |
| Glutathion | 356,16000 | thione : 400,14700 |
| DL-Pénicillamine | 354,16000 | adduit : 358,15000 |
| N-Acétyl-L-cystéine | 358,15000 | hydrolyse:329,10500 |
| Mercaptosuccinate | 362,15000 | hydrolyse:329,10800 |
| Cystéine | 352,18500 | adduit : 356,18400 |
| Homocystéine | 352,15000 | adduit : 356,14000 |
| 2-Mercaptopyridine | 345,14500 | hydrolyse:327,13000 |
| Diéthyldithiocarbamate | 279,13000 | hydrolyse:329,10400 |
| Captopril | 359,14000 | hydrolyse:329,10300 |
| Thio-β-D-glucose | 341,14000 | hydrolyse:329,10700 |
| Cystéinylglycine | 350,16000 | adduit:355,13000 |
| Coenzyme A | 359,16000 | hydrolyse:329,8000 |
| Sérum Albumine bovine | (*) 345,12500 | (**) |

(*) Il ne se forme que 0,50 équivalent d'adduit (voir référence 21).

(**) Le spectre d'absorption est similaire à celui obtenu avant alcalinisation.

La quantification des mercaptans totaux est réalisée comme indiqué plus haut.

La sensibilité du dosage a été déterminée à partir d'une gamme étalon en certains mercaptans physiologiquement importants. Elle est, par exemple pour le GSH, égale à 0,017 $UA.l.\mu mole^{-1}$ avec le réactif BXT03015, comme cela ressort de l'examen de la figure 1 des dessins annexés (UA = unités d'absorbance).

La figure 1 représente les variations de l'absorbance à 356 nm de l'adduit formé entre le réactif BXT 03015 et le GSH, la N-acétylcystéine, la cystéine et la cystéinyl-glycine, respectivement, en fonction de la concentration micromolaire en mercaptans.

On constate que l'absorbance varie de façon linéaire en fonction de cette concentration et que la sensibilité du dosage est égale à 0,017 $UA.l.\mu mole^{-1}$, pour le GSH.

Dosage du glutathion réduit :

Le même mélange réactionnel, utilisé précédemment pour le dosage des mercaptans totaux, est réutilisé pour le dosage du glutathion réduit. Ce mélange est ajusté à une valeur de pH = 13,5 par l'addition d'une solution alcaline concentrée, comme par exemple une solution de soude à 30 %, à la température de travail choisie, par exemple 25°C. Après 15-20 minutes d'incubation dans ces mêmes conditions, l'absorbance de l'unique produit de β-élimination de l'adduit entre le GSH et le réactif BXT, est mesurée à la longueur d'onde définie pour le réactif BXT considéré, par exemple 400 nm pour le réactif BXT03015. Le tableau II qui suit contient les données spectrophotométriques obtenues avec quelques réactifs selon l'invention et le GSH.

TABLEAU II

Données spectrales des chromophores obtenus par réaction entre le GSH et quelques réactifs BXT dont la préparation est décrite dans la partie expérimentale qui suit, avant et après alcalinisation

| | | AVANT | APRES |
|---|---|---|---|
| | | alcalinisation | |
| | REACTIF BXT | Adduit | Thione |
| N° | λmax(nm) | λmax(nm) | λmax(nm) |
| | εm($l.mole^{-1}.cm^{-1}$) | εm($l.mole^{-1}.cm^{-1}$) | εm($l.mole^{-1}.cm^{-1}$) |
| BXT 03015 (ex.1) | 315<br>8000 | 356<br>16000 | 400<br>14700 |
| BXT 03016 (ex.2) | 321<br>12000 | 339<br>15000 | 381<br>16000 |
| BXT 03034 (ex.3) | 319<br>9000 | 346<br>15000 | 388<br>18000 |
| BXT 03065 (ex.5) | 326<br>9000 | 343<br>17000 | 393<br>20000 |
| BXT 03069 (ex.9) | 312<br>5400 | 414<br>6000 | 520<br>2500 |

La sensibilité du dosage est déterminée à partir d'une gamme standard de concentrations en GSH, et est égale par exemple à 0,010 $UA.l.\mu mole^{-1}$ avec le réactif BXT03015, comme cela résulte de la figure 3 des dessins annexés.

La figure 2 représente les variations de l'absorbance ou densité optique (D.O.) à 356 nm de l'adduit formé entre le GSH et le réactif BXT03015, avant alcalinisation, en fonction de la concentration (micromolaire) en GSH.

On constate que l'absorbance varie de façon linéaire en fonction de la concentration en GSH. Cette figure montre en outre la sensibilité du dosage.

La figure 3 représente les variations de l'absorbance ou densité optique (D.O.) à 400 nm de la thione formée à partir de l'adduit GSH/réactif BXT03015, après alcalinisation, en fonction de la concentration (micromolaire) en GSH.

On constate que l'absorbance varie de façon linéaire en fonction de la concentration en GSH. Cette figure montre en outre la sensibilité du dosage.

L'invention a en outre pour objet un nécessaire ou kit pour la mise en oeuvre des procédés selon l'invention définis ci-dessus, caractérisé en ce qu'il comprend essentiellement, en tant que réactif, un composé de formule générale I.

L'invention sera expliquée plus en détail à l'aide des exemples non limitatifs donnés dans la partie expérimentale qui suit.

PARTIE EXPERIMENTALE

I. Exemples de synthèse de réactifs de formule générale I:

**Exemple 1: Tétrafluoroborate de 4-chloro-7-trifluorométhyl-1-méthylquinoléinium (BXT03015)**.

Dans un ballon de 50 ml muni d'une agitation, d'un thermomètre, et sous courant d'azote, on dissout sous agitation 2,15 mmoles de 4-chloro-7-trifluorométhyl-quinoléine (ALDRICH) dans 5 ml d'acétonitrile. A 10°C, une solution de 4,30 mmoles de tétrafluoroborate de triméthyloxonium (LANCASTER) dans 4 ml d'acétonitrile est ajoutée en 10 minutes. L'agitation est maintenue 1 heure et le solvant est évaporé sous vide. Le résidu brut est repris par 50 ml d'éther terbutylméthylique, essoré et séché sous vide pendant 2 heures.

Le produit brut est purifié par HPLC préparative sur colonne ($C_8$, 5 µm, L = 250 mm, DI = 10,5 mm), en éluant par un mélange acétonitrile-eau (20/80). Après lyophilisation, le produit désiré (1,7 mmoles) est obtenu. Rendement 77 %.

Caractéristiques physiques :

*RMN $^1H$ (200 MHz, $CD_3COCD_3$) :

$CH_3$-$N^+$: 5,00 ppm (s, 3H); $H_6$ : 8,45 ppm (dd, 1H, J = 1,9-9,5 Hz) ; $H_3$: 8,60 ppm (d, 1H, J = 7,14 Hz); $H_5$ : 8,95 ppm (d, 1H, J = 9,50 Hz); $H_8$ : 9,10 ppm (s, 1H); $H_2$: 9,70 ppm (d, 1H, J = 7,14 Hz).

*SM : (FAB, glycérol-NOBA) ion positif : $M^+$ : 246
ion négatif : $M^-$ : 87.

FAB = bombardement par atomes rapides (Fast Atom Bombardment)
NOBA = alcool méta-nitrobenzylique

| *Microanalyse : | | | | |
|---|---|---|---|---|
| Calculé (%) : | C = 39,6 ; | H = 2,4 ; | N = 4,2 ; | Cl = 10,6 |
| Trouvé (%) : | C = 39,4 ; | H = 2,2 ; | N = 4,0 ; | Cl = 10,2. |

**Exemple 2 : Tétrafluoroborate de 4-chloro-1,2-diméthylquinoléinium (BXT03016).**

Dans un ballon de 50 ml muni d'une agitation, d'un thermomètre, et sous courant d'azote, on dissout sous agitation 2,0 mmoles de 4-chloro-2-méthylquinoléine (ALDRICH) dans 5 ml d'acétonitrile. A 10°C, une solution de 4,0 mmoles de tétrafluoroborate de triméthyloxonium (LANCASTER) dans 4 ml d'acétonitrile est ajoutée en 10 minutes. L'agitation est maintenue 1 heure et le solvant est évaporé sous vide. Le résidu brut est repris par 50 ml d'éther terbutylméthylique, essoré et séché sous vide pendant 2 heures.

Le produit brut est purifié par HPLC préparative sur colonne (C8, 5 µm, L = 250 mm, DI = 10,5 mm), en éluant par un mélange acétonitrile-eau (20/80). Après lyophilisation, le produit désiré (0,8 mmole) est obtenu.
Rendement : 38 %.
Caractéristiques physiques :

*RMN $^1H$ (200 MHz, $CD_3COCD_3$) :

$CH_3$ : 3,2 ppm (s, 3H); $CH_3$-$N^+$ : 4,70 ppm (s, 3H); $H_7$ : 8,10 ppm (dd, 1H, J = 8-6,5 Hz); $H_6$ : 8,30 ppm (dd, 1H, J = 8-6,5 Hz); $H_3$: 8,40 ppm (s, 1H); $H_5$ : 8,60 ppm (d, 1H, J = 8 Hz); $H_8$ : 8,70 ppm (d, 1H, J = 8 Hz).

*SM: (FAB, glycérol-NOBA) ion positif : $M^+$ : 192
ion négatif : $M^-$ : 87.

| *Microanalyse : | | | | |
|---|---|---|---|---|
| Calculé (%) : | C = 47,2 ; | H = 3,9 ; | N = 5,0 ; | Cl = 12,7 |
| Trouvé (%) : | C = 47,3 ; | H = 3,9 ; | N = 4,9 ; | Cl = 13,0. |

**Exemple 3 : Tétrafluoroborate de 4-chloro-1-méthylquinoléinium (BXT03034)**.

Dans un ballon de 100 ml muni d'une agitation, d'un thermomètre et sous courant d'azote, on dissout sous agitation 3,17 mmoles de 4-chloroquinoléine (ALDRICH) dans 10 ml d'acétonitrile. A température ambiante, une solution de 6,30 mmoles de tétrafluoroborate de triméthyloxonium (LANCASTER) dans 4 ml d'acétonitrile est ajoutée en 10 minutes. L'agitation est maintenue 30 minutes et le solvant est évaporé sous vide. Le résidu brut est repris par 10 ml de 2-propanol et agité 10 minutes. Le précipité est filtré, lavé par 50 ml d'éther terbutylméthylique, essoré et séché sous vide pendant 2 heures.

Après recristallisation dans un mélange éther terbutylméthylique/2-propanol (1/1), le produit désiré (1,5 mmoles) est obtenu. Rendement 84 %.

Caractéristiques physiques :

*RMN $^1H$ (200 MHz, $CD_3COCD_3$) :

$CH_3$-$N^+$ : 4,45 ppm (s, 3H); $H_3$ : 8,20 ppm (d, 1H, J = 7,5 Hz); $H_6$ et $H_7$ : 8,40 ppm (m, 2H); $H_5$ et $H_8$ : 8,70 ppm (dd, 2H, J = 8-1,8 Hz); $H_2$: 9,5 ppm (d, 1H, J = 7,5 Hz).

*SM: (FAB, glycérol-NOBA) ion positif : $M^+$: 178
Ion négatif : $M^-$ : 87.

**Exemple 4 : <u>Tétrafluoroborate de 7-chloro-1,2-diméthylquinoléinium (BXT03064)</u>.**

Dans un ballon de 50 ml muni d'une agitation, d'un thermomètre, et sous courant d'azote, on dissout sous agitation 2,0 mmoles de 7-chloro-2-méthylquinoléine (ALDRICH) dans 5 ml d'acétonitrile. A 10°C, une solution de 2,60 mmoles de tétrafluoroborate de triméthyloxonium (LANCASTER) dans 4 ml d'acétonitrile est ajoutée en 10 minutes. L'agitation est maintenue 1 heure 30 et le solvant est évaporé sous vide. Le résidu brut est repris par 50 ml d'éther terbutylméthylique, essoré et séché sous vide pendant 2 heures.

Après recristallisation dans un mélange éther terbutylméthylique/2-propanol (1/1), le produit désiré (1,5 mmoles) est obtenu. Rendement : 76 %.

Caractéristiques physiques :

*RMN $^1H$ (200 MHz, $CD_3COCD_3$) :

$CH_3$: 3,20 ppm (s, 3H); $CH_3$-$N^+$ : 4,65 ppm (s, 3H); $H_6$ : 8,00 ppm (dd, 1H, J = 8,8-1,6 Hz); $H_3$ : 8,12 ppm (d, 1H, J = 8 Hz); $H_5$ : 8,45 ppm (d, 1H, J = 8,8 Hz) ; $H_8$ : 8,72 ppm (d, 1H, J = 1,8 Hz); $H_4$ : 9,12 ppm (d, 1H, J = 8 Hz).

*SM : (FAB, glycérol-NOBA) ion positif : $M^+$ : 192
ion négatif : $M^-$ : 87.

| *Microanalyse : | | | | |
|---|---|---|---|---|
| Calculé (%) : | C = 47,2 ; | H = 3,9 ; | N = 5,0 ; | Cl = 12,7 |
| Trouvé (%) : | C = 47,0 ; | H = 3,8 ; | N = 5,1 ; | Cl = 12,5. |

**Exemple 5 : <u>Tétrafluoroborate de 4,7-dichloro-1-méthylquinoléinium (BXT03065)</u>.**

Dans un ballon de 50 ml muni d'une agitation, d'un thermomètre, et sous courant d'azote, on dissout sous agitation 2,0 mmoles de 4,7-dichloroquinoléine (ALDRICH) dans 5 ml d'acétonitrile. A 10°C, une solution de 2,60 mmoles de tétrafluoroborate de triméthyloxonium (LANCASTER) dans 4 ml d'acétonitrile est ajoutée en 10 minutes. L'agitation est maintenue 1 heure et le solvant est évaporé sous vide. Le résidu brut est repris par 50 ml d'éther terbutylméthylique, essoré et séché sous vide pendant 2 heures.

Après recristallisation dans un mélange éther terbutylméthylique/2-propanol (1/1), le produit désiré (1,4 mmoles) est obtenu. Rendement : 70 %.

Caractéristiques physiques :

*RMN $^1H$ (200 MHz, $D_2O$) :

$CH_3$-$N^+$: 7 ppm (s, 3H); $H_6$ : 10,49 ppm (dd, 1H, J = 9-2,2 Hz); $H_3$: 10,58 ppm (d, 1H, J = 6,7 Hz); $H_8$ : 10,94 ppm (d, 1H, J = 2,1 Hz); $H_5$ : 11,2 ppm (d, 1H, J = 9 Hz); $H_2$: 11,56 ppm (d, 1H, J = 6,5 Hz).

*SM: (FAB, glycéro-NOBA) ion positif : $M^+$ : 212
ion négatif : $M^-$ : 87.

| *Microanalyse : | | | | |
|---|---|---|---|---|
| Calculé (%) : | C = 40,0 ; | H = 2,6 ; | N = 4,6 ; | Cl = 23,6 |
| Trouvé (%) : | C = 41,1 ; | H = 2,4 ; | N = 4,8 ; | Cl = 23,6. |

**Exemple 6 : Tétrafluoroborate de 5-chloro-8-hydroxy-1-méthylquinoléinium (BXT03066)**.

Dans un ballon de 50 ml muni d'une agitation, d'un thermomètre, et sous courant d'azote, on dissout sous agitation 2,0 mmoles de 5-chloro-8-hydroxyquinoléine (ALDRICH) dans 5 ml d'acétonitrile. A 10°C, une solution de 2,40 mmoles de tétrafluoroborate de triméthyloxonium (LANCASTER) dans 4 ml d'acétonitrile est ajoutée en 10 minutes. L'agitation est maintenue 30 minutes et le solvant est évaporé sous vide. Le résidu brut est repris par 50 ml d'éther terbutylméthylique, essoré et séché sous vide pendant 2 heures.

Après recristallisation dans un mélange éther terbutylméthylique/2-propanol (1/1), le produit désiré (1,4 mmoles) est obtenu. Rendement 70 %.

Caractéristiques physiques :

*RMN $^1H$ (200 MHz, $CD_3COCD_3$) :

$CH_3$-$N^+$: 5,05 ppm (s, 3H); $H_7$ : 7,65 ppm (d, 1H, J = 8,6 Hz); $H_6$ : 7,95 ppm (d, 1H, J = 8,6 Hz); $H_3$: 8,20 ppm (dd, 1H, J = 5,7-2,3 Hz); $H_4$ : 9,35 ppm (d, 1H, J = 5,7 Hz); $H_2$: 9,40 ppm (d, 1H, J = 2,3 Hz).

*SM: (FAB, glycérol-NOBA) ion positif : $M^+$ : 194
ion négatif : $M^-$ : 87.

**Exemple 7 : Tétrafluoroborate de 5-chloro-8-benzyloxy-1-méthylquinoléinium (BXT03063)**.

**5-Chloro-8-benzyloxyquinoléine :**

Dans un ballon de 50 ml muni d'une agitation et d'un thermomètre, on dissout sous agitation 10,0 mmoles de 5-chloro-8-hydroxyquinoléine (ALDRICH) dans 20 ml de DMF sec. Puis on ajoute 11 mmoles de carbonate de potassium et 10 % en poids d'iodure de potassium. Une solution de 11 mmoles de chlorure de benzyle (JANSSEN) dans 5 ml de DMF est ajoutée goutte à goutte en 5 minutes à température ambiante et sous agitation. Après 18 heures de réaction à 50°C, le mélange réactionnel, additionné de 100 ml d'eau, est extrait par 4x50 ml d'éther terbutylméthylique. Les phases organiques sont rassemblées, puis lavées par 3x50 ml d'HCl 1N. Les phases aqueuses sont rassemblées, alcalinisées à pH = 9 et extraites par 2x50 ml d'acétate d'éthyle. Après séchage de la phase organique sur $MgSO_4$, le solvant est évaporé sous pression réduite. Le produit désiré est obtenu après recristallisation dans de l'éthanol. Rendement:79,5 %.

Caractéristiques physiques :

*RMN $^1H$ (200 MHz, $CD_3COCD_3$) :

$CH_2O$ : 5,35 ppm (s, 2H); 7,22-7,45 ppm (m, 4H); 7,55-7,70 ppm (m, 4H); $H_4$ : 8,53 ppm (d, 1H, J = 5,2 Hz); $H_2$: 8,95 ppm (d, 1H, J = 2,1 Hz).

**Tétrafluoroborate de 5-chloro-8-benzyloxy-1-méthylquinoléinium (BXT03063) :**

Dans un ballon de 50 ml muni d'une agitation, d'un thermomètre, et sous courant d'azote, on dissout sous agitation 10,0 mmoles de 5-chloro-8-benzyloxyquinoléine dans 5 ml d'acétonitrile. A 10°C, une solution de 23,0 mmoles de tétrafluoroborate de triméthyloxonium (LANCASTER) dans 4 ml d'acétonitrile est ajoutée en 10 minutes. L'agitation est maintenue 1 heure et le solvant est évaporé sous vide. Le résidu brut est repris par 50 ml d'éther terbutylméthylique, essoré et séché sous vide pendant 2 heures.

Le produit brut est purifié par HPLC préparative sur colonne (C8, 5 µm, L = 250 mm, DI = 10,5 mm), en éluant par un mélange acétonitrile-eau (20/80) . Après lyophilisation, le produit désiré (2,0 mmoles) est obtenu. Rendement : 21 %.

Caractéristiques physiques :

*RMN $^1H$ (200 MHz, $CD_3COCD_3$) :

$CH_3$-$N^+$: 5,00 ppm (s, 3H); $CH_20$ : 5,55 ppm (s, 2H); $H_{2'}$, $H_{4'}$ et $H_{6'}$ : 7,45 ppm (m, 3H); $H_{3'}$ et $H_{5'}$ : 7,75 ppm (m, 2H); $H_7$ : 7,95 ppm (d, 1H, J = 10 Hz); $H_6$ : 8,15 ppm (d, 1H, J = 10 Hz), $H_3$ : 8,35 ppm (t, 1H, J = 11,2 Hz); $H_2$ et $H_4$ : 9,5 ppm (d, 2H, J = 11,4 Hz).

*SM : (FAB, glycérol-NOBA) ion positif : $M^+$ : 284
ion négatif : $M^-$ : 87.

**Exemple 8 : Tétrafluoroborate de 5-fluoro-1-méthylquinoléinium (BXT03068)** .

**5-Fluoroquinoléine :**

Dans un tricol de 100 ml muni d'une agitation et d'un thermomètre, on dissout à température ambiante 13,7 mmoles

de 5-aminoquinoléine (ALDRICH) dans 20 ml d'éthanol. Une solution d'acide tétrafluoroborique à 34 % est ajoutée en 5 minutes. Le mélange réactionnel est refroidi à -5°C et 36,0 mmoles de nitrite d'isoamyle (JANSSEN) sont ajoutées en 10 minutes à cette température. A la fin de l'addition, le milieu réactionnel est maintenu à 0°C sous agitation pendant 1 heure. La suspension obtenue est filtrée, le solide est lavé par 3x20 ml d'éthanol absolu et 3x20 ml d'éther éthylique, puis séché sous vide pendant 18 heures. Le sel de diazonium ainsi obtenu avec un rendement de 85,7 % est utilisé tel quel pour l'étape suivante.

Dans un tricol de 250 ml muni d'une agitation, d'un thermomètre et d'un réfrigérant ascendant, lui-même équipé d'un bulleur à huile, le sel de diazonium obtenu précédemment est mis en suspension dans 100 ml d'heptane anhydre et chauffé au reflux pendant 3 heures, sous agitation. Le mélange réactionnel est refroidi et additionné d'une solution de soude 1N (100 ml). La phase organique est décantée, lavée par 2x100 ml de cette même solution de soude, puis par 2x100 ml d'eau saturée en NaCl et séchée sur $MgSO_4$. Le solvant est évaporé sous vide à l'évaporateur rotatif.

Le produit désiré est purifié par chromatographie sur colonne de silice Geduran® [MERCK, 40-63µm (230-400 mesh)]: éluant $CH_2Cl_2$. Rendement : 35 %.

Caractéristiques physiques :

*RMN $^1H$ (200 MHz, $CDCl_3$) :

$H_6$ : 7,26-7,40 ppm (ddd, 1H, J = 1-6,8-9,7 Hz); $H_3$ : 7,54-7,64 ppm (dd, 1H, J = 4,8-10 Hz); $H_7$ : 7,67-7,79 ppm (ddd, 1H, J = 4-6,8-8 Hz); $H_8$ : 7,88 ppm (dd, 1H, J = 1-8 Hz); $H_4$: 8,45 ppm (dd, 1H, J = 2-10 Hz); $H_2$ : 8,95 ppm (dd, 1H, J = 2-4,8 Hz).

*SM: (IE, 70eV) $M^+$: 147(100); 120(21); 99(10); 74(14). IE = impact électronique

**Tétrafluoroborate de 5-fluoro-1-méthylquinoléinium (BXT03068) :**

Dans un ballon de 50 ml muni d'une agitation, d'un thermomètre, et sous courant d'azote, on dissout sous agitation 10,0 mmoles de 5-fluoroquinoléine dans 5 ml d'acétonitrile. A 10°C, une solution de 23,0 mmoles de tétrafluoroborate de triméthyloxonium (LANCASTER) dans 4 ml d'acétonitrile est ajoutée en 10 minutes. L'agitation est maintenue 1 heure et le solvant est évaporé sous vide. Le résidu brut est repris par 50 ml d'éther terbutylméthylique, essoré et séché sous vide pendant 2 heures.

Le produit désiré est recristallisé dans un mélange éther terbutylméthylique/2-propanol (1/1).

Rendement : 50 %.

Caractéristiques physiques :

*RMN $^1H$ (200 MHz, $CD_3COCD_3$) :

$CH_3$-$N^+$ : 4,90 ppm (s, 3H); $H_3$ : 7,85 ppm (dd, 1H, J = 9,5-12 Hz); $H_6$ et $H_7$ : 8,25-8,40 ppm (m, 2H); $H_8$ : 8,48 ppm (d, 1H, J = 12,3 Hz); $H_4$ : 9,45 ppm (d, 1H, J = 12 Hz);$H_2$ : 9,65 ppm (d, 1H, J = 9,5 Hz).

*SM: (IE, 70eV) $M^+$ : 162(80); 147(100); 120(48); 99(21); 74(20).

**Exemple 9 : <u>Fluorosulfonate de 5-fluoro-8-nitro-1-méthylquinoléinium (BXT03069)</u> .**

**5-Fluoro-8-nitroquinoléine** :

Dans un tricol de 100 ml, muni d'une agitation et d'un thermomètre, on introduit une solution de 20,0 mmoles de 5-fluoroquinoléine dans 10 ml d'acide sulfurique concentré (d = 1,84). En maintenant la température à 0°C, 5 ml d'acide nitrique fumant sont ajoutés goutte à goutte, sous agitation. Le mélange réactionnel est maintenu à 20°C pendant 10 minutes, puis versé lentement sur de la glace (100 g). Le mélange réactionnel est amené à pH = 8 par addition lente de soude concentrée. Le précipité ainsi obtenu est extrait par 100 ml d'acétate d'éthyle. La phase organique est lavée par 2x50 ml d'eau et séchée sur $MgSO_4$. Le solvant est évaporé sous pression réduite.

Le produit désiré est purifié par chromatographie sur colonne de silice Geduran®: éluant $CH_2Cl_2$. Rendement : 67 %.

Caractéristiques physiques :

*RMN $^1H$ (200 MHz, $CDCl_3$) :

$H_6$: 7,27 ppm (dd, 1H, J = 8,58-8,64 Hz); $H_3$ : 7,62 ppm (dd, 1H, J = 8,60-4,20 Hz); $H_7$ : 8,10 ppm (dd, 1H, J = 8,56-5,22 Hz), $H_4$ : 8,48 ppm (dd, 1H, J = 2,5-8,6 Hz); H2 : 9,12 ppm (dd, 1H, J = 2,5-4,2 Hz).

*SM : (IE, 70eV) $M^+$: 192(100), 176(16), 162(47), 146(33), 134(98), 126(43), 119(21), 107(36), 99(44).

**Fluorosulfonate de 5-fluoro-8-nitro-1-méthylquinoléinium (BXT03069) :**

Dans un tricol de 100 ml muni d'une agitation, d'un thermomètre, et sous courant d'azote, on dissout sous agitation 2,5 mmoles de 5-fluoro-8-nitroquinoléine dans 10 ml de glyme sec. A température ambiante, on ajoute en 3 minutes, 1 ml de fluorosulfonate de méthyle (10,0 mmoles; ALFA VENTRON). Le mélange réactionnel est agité 1 heure à 60°C.

Après retour à température ambiante, le précipité obtenu est essoré et lavé par 3x10 ml d'éther terbutylméthylique puis séché sous vide. Le produit désiré est obtenu avec un rendement de 46 %.
Caractéristiques physiques :
*RMN $^1$H (200 MHz, $CD_3COCD_3$) :
CH3-$N^+$ : 4,70 ppm (s, 3H); $H_6$ : 8,10 ppm (dd, 1H, J = 8,46-8,56 Hz) ; $H_3$: 8,58 ppm (dd, 1H, J = 8,72-5,86 Hz) ; $H_7$: 8,90 ppm (dd, 1H, J = 8,72-5,28 Hz); $H_4$ : 9,66 ppm (d, 1H, J = 8,8 Hz); $H_2$ : 9,88 ppm (d, 1H, J = 5,86 Hz).

*SM: (FAB, glycérol-NOBA) ion positif : $M^+$ : 207
ion négatif : $M^-$ : 404
(2 $M^-$/1 $M^+$)
$M^-$ : 99.

**Exemple 10 : Tétrafluoroborate de 5-nitro-1-méthylquinoléinium (BXT03070) .**

Dans un ballon de 50 ml muni d'une agitation, d'un thermomètre, et sous courant d'azote, on dissout sous agitation 5,74 mmoles de 5-nitroquinoléine (ALDRICH) dans 10 ml d'acétonitrile. A 25°C, une solution de 6,4 mmoles de tétrafluoroborate de triméthyloxonium (LANCASTER) dans 4 ml d'acétonitrile est ajoutée en 10 minutes. L'agitation est maintenue 1/2 heure et le solvant est évaporé sous vide. Le résidu brut est repris par un mélange éther terbutylméthylique/2-propanol (1/1), essoré et séché sous vide pendant 2 heures.
Le produit désiré est recristallisé dans un mélange éther terbutylméthylique/2-propanol (1/1).
Rendement : 89 %.
Caractéristiques physiques :
*RMN $^1$H (200 MHz, $D_20$) :
CH3-$N^+$ : 4,70 ppm (s, 3H); $H_3$ : 8,20 ppm (dd, 1H, J = 9,6-5,6 Hz); $H_7$ : 8,33 ppm (t, 1H, J = 8,6 Hz); $H_6$ et $H_8$ : 8,74 ppm (t, 2H, J = 8,6 Hz); $H_4$ :9,37 ppm (d, 1H, J = 5,6 Hz); $H_2$: 9,64 ppm (d, 1H, J = 9,6 Hz).

*SM: (FAB, glycérol-NOBA) ion positif : $M^+$ : 189
ion négatif : $M^-$ : 87.

**Exemple 11 : Fluorosulfonate de 6-chloro-7-trifluorométhyl-1-méthylquinoléinium et fluorosulfonate de 6-chloro-5-trifluorométhyl-1-méthylquinoléinium :**

**6-Chloro-7-trifluorométhylquinoléine et 6-chloro-5-trifluorométhylquinoléine :**

Dans un tricol de 100 ml, muni d'une agitation et d'un thermomètre, on introduit 20 mmoles de 5-amino-2-chloro-1-trifluorométhylbenzène (ALDRICH) et 14 mmoles d'oxyde d'arsenic (ALDRICH) dans 80 ml de glycérol anhydre (SIGMA). 2,15 ml d'acide sulfurique concentré (d = 1,84) sont ajoutés goutte à goutte, à température ambiante. Le mélange réactionnel est placé sous vide [2666 Pa (20 mm de Hg)], tandis que la température est amenée progressivement à 110°C. L'agitation est maintenue à cette température pendant 4 heures. Puis le mélange réactionnel est ramené à la pression atmosphérique, et une seconde addition d'acide sulfurique concentré (d = 1,84; 1 ml) est effectuée. L'élimination de l'eau est alors reprise sous agitation vigoureuse pendant 4 heures sous vide [2666 Pa (20 mm de Hg)] à une température ne dépassant jamais 120°C. Après retour du milieu réactionnel à une température de 100°C, 50 ml d'eau sont ajoutés lentement. La solution résultante, revenue à température ambiante et dont le pH est rendu basique par addition d'une solution concentrée d'ammoniaque (d = 0,89; 5 ml; PROLABO), est extraite par de l'acétate d'éthyle (2x100 ml). Les phases organiques sont rassemblées, séchées sur sulfate de magnésium puis le solvant est évaporé sous vide. Le résidu solide est finalement chromatographié sur colonne de silice Geduran® [MERCK; 40-63 µm (200-400 mesh)] à l'aide d'un mélange éluant : chlorure de méthylène/pentane (4/1).
6-chloro-7-trifluorométhylquinoléine : rendement = 24 %.
Caractéristiques physiques :
*pF = 137-139°C.
*RMN $^1$H (200 MHz, $CDCl_3$) :
$H_3$: 7,53 ppm (dd, 1H, J = 4,18-8,40 Hz); $H_5$ : 7,96 ppm (s, 1H); $H_4$ : 8,12 ppm (d, 1H, J = 7,40 Hz); $H_8$ : 8,48 ppm (s, 1H); $H_2$ : 8,99 ppm (dd, 1H, J = 1,38-2,78 Hz).
*SM (IE, 70eV) : 233(30); 231(100); 212(10); 196(20).
6-chloro-5-trifluorométhylquinoléine : rendement = 8 %.
Caractéristiques physiques :
*RMN $^1$H (200 MHz, $CDCl_3$) :
$H_3$: 7,40 ppm (dd, 1H, J = 4,20-8,96 Hz); $H_7$ : 7,58 ppm (d, 1H, J = 9,16 Hz); $H_8$ : 8,03 ppm (d, 1H, J = 8,94 Hz);

$H_4$ : 8,45 ppm (d, 1H, J = 9,00 Hz); $H_2$: 8,85 ppm (d, 1H, J = 2,94 Hz).
*SM (IE, 70eV) : 233(30); 231(100); 212(10); 196(20); 179(15).

**Fluorosulfonate de 6-chloro-5-trifluorométhyl-1-méthylquinoléinium (BXT03071) :**

Dans un ballon de 50 ml, muni d'une agitation et sous courant d'azote, on introduit 0,6 mmole de 6-chloro-5-trifluorométhylquinoléine dans 5 ml d'éther diméthylique de l'éthylène glycol puis goutte à goutte, sous agitation, 0,2 ml (2,4 mmoles) de fluorosulfonate de méthyle (ALFA). Après 1 heure de chauffage à 50°C puis retour à température ambiante, la solution résultante est additionnée de 10 ml d'éther éthylique. Le précipité formé est filtré, lavé à l'éther éthylique (2x5 ml) puis séché à l'étuve sous vide, à 40°C, sur anhydride phosphorique.

Le produit désiré (poudre blanche) est isolé avec un rendement de 72 %.

Caractéristiques physiques :
*pF = 149-151°C.
*RMN $^1$H (200 MHz), $CD_3SOCD_3$) :
$CH_3$-$N^+$ : 4,70 ppm (s, 3H); $H_3$ : 8,35 ppm (dd, 1H, J = 5,78-9,12 Hz); $H_7$ : 8,51 ppm (d, 1H, J = 9,8 Hz); $H_8$ : 8,86 ppm (d, 1H, J = 9,9 Hz), $H_4$ : 9,38 ppm (d, 1H, J = 9,06 Hz); $H_2$ : 9,64 ppm (d, 1H, J = 5,48 Hz).

*SM (FAB ; glycérol-NOBA) ion positif : $M^+$: 345
ion négatif : $M^-$ : 99.

**Fluorosulfonate de 6-chloro-7-trifluorométhyl-1-méthylquinoléinium (BXT03072) :**

Le mode opératoire mis en oeuvre pour l'obtention de ce composé est identique à celui du composé précédent en remplaçant la 6-chloro-5-trifluorométhylquinoléine par la 6-chloro-7-trifluorométhylquinoléine. Le produit désiré est obtenu avec un rendement de 80 %.

Caractéristiques physiques :
*pF = 187-189°C.
*RMN $^1$H (200 MHz, $CD_3SOCD_3$) :
$CH_3$-$N^+$: 4,78 ppm (s, 3H); $H_3$: 8,38 ppm (dd, 1H, J = 6,0-9,3 Hz); $H_5$ : 8,90 ppm (d, 1H); $H_8$ : 8,98 ppm (s, 1H); $H_4$ : 9,25 ppm (d, 1H, J = 7,5 Hz); $H_2$: 9,64 ppm (d, 1H, J = 7,0 Hz).

*SM (FAB ; glycérol-NOBA) ion positif : $M^+$: 345
ion négatif : $M^-$ : 99.

II. Exemples d'applications :

Les protocoles opératoires décrits ci-après sont des exemples d'applications du procédé selon l'invention au dosage des mercaptans non protéiques et du glutathion réduit sur un lysat érythrocytaire, à l'aide du réactif BXT03015.

Un exemple de coffret de réactifs est décrit ci-dessous. Ce coffret ou kit de réactifs selon l'invention doit être stocké à une température comprise entre 0 et 4°C.

Description des réactifs :

**T** : Tampon phosphate 50 mM, pH = 7,3, contenant du DTPA 1 mM.
(On le laisse s'équilibrer à l'air et à 25°C avant utilisation).
**S1** : Solution d'acide métaphosphorique à 5 %.
(Doit être maintenue entre 0 et 4°C pour la préparation de l'échantillon).
**S2** : Solution de soude à 30 %.
(On la laisse s'équilibrer à l'air et à 25°C).
**R1** : Réactif BXT sous forme solide.
(On prépare extemporanément une solution-mère $10^{-2}$ M dans le tampon **T**).
**E** : GSH sous forme solide.

Préparation de l'échantillon :

- on centrifuge 2 ml de sang total à 4°C et 3000 tr/min pendant 10 minutes ;
- on élimine le surnageant ;
- on reprend le culot érythrocytaire avec 4 fois son volume de solution **S1** refroidie entre 0 et 4°C ;

- on mélange ;
- on centrifuge à 4°C et 3000 tr/min pendant 10 minutes.

Le surnageant ainsi obtenu correspondant à un lysat érythrocytaire, dont les protéines ont été précipitées, dilué au 1/5e, constituera l'échantillon à doser (il doit être maintenu entre 0 et 4°C pour les mesures).

1. Dosage des mercaptans totaux :

Protocole du dosage :

Les mesures sont effectuées à 25°C dans un spectrophotomètre dont les cuves de mesure sont thermostatées.

Le mélange réactionnel correspondant à chaque dosage est préparé extemporanément selon le protocole suivant, pour des cuves de 1 ml :

- on mélange : 100 µl de l'échantillon à doser et
  800 µl de solution **T** ;
- on laisse incuber pendant 1 minute à 25°C ;
- on déclenche la réaction en ajoutant 100 µl de solution-mère de **R1** $10^{-2}$ M dans le tampon **T** ; et
- on laisse incuber pendant 5 minutes à 25°C.

La mesure d'absorbance (A) est effectuée à 356 nm dans le cas où **R1** est le composé BXT03015, dans des cuves de 1 cm de trajet optique, contre de l'air.

Le GSH présent dans le coffret de réactifs (E) peut être utilisé pour définir une courbe d'étalonnage, si nécessaire.

Expression des résultats :

Dans les conditions de ce dosage, la concentration en mercaptans totaux est déterminée selon la formule :

$$[\text{mercaptans totaux}] = (A/16200) \times 50$$

dans laquelle A est l'absorbance mesurée.

Remarque : Le coefficient d'extinction molaire utilisé ici (16200 $l.mole^{-1}cm^{-1}$) est une moyenne des coefficients d'extinction molaire à 356 nm obtenus pour les adduits formés entre le réactif BXT03015 et les principaux mercaptans physiologiquement importants décrits dans le Tableau I qui précède. L'erreur relative est égale, dans ces conditions, à ± 9 %.

Dans ce cas, on obtient une mesure de la concentration en mercaptans totaux dans l'échantillon exprimée en moles/l.

2. Dosage du glutathion réduit :

Protocole du dosage :

Les mesures sont effectuées à 25°C dans un spectrophotomètre dont les cuves de mesure sont thermostatées.

Le mélange réactionnel correspondant à chaque dosage est préparé extemporanément selon le protocole suivant, pour des cuves de 1 ml :

- à la solution ayant servi à doser les mercaptans totaux, on ajoute 50 µl de solution **S2** ; et
- on laisse incuber pendant 15 minutes à 25°C.

La mesure d'absorbance est effectuée à 400 nm dans le cas où **R1** est le composé BXT03015, dans des cuves de 1 cm de trajet optique, contre de l'air.

Expression des résultats :

Dans les conditions de ce dosage, la concentration en GSH est déterminée selon la formule :

$$[GSH] = (A'/14700) \times 50$$

dans laquelle A' représente l'absorbance mesurée.

Dans ce cas, on obtient une mesure de la concentration en glutathion réduit (GSH) exprimée en moles/l.

Une solution de GSH (réactif **E**) à $10^{-2}$ mole/l dans de l'acide métaphosphorique à 5 % (solution **S1**) peut être préparée extemporanément et servir pour une courbe d'étalonnage, si nécessaire.

<u>BIBLIOGRAPHIE</u>


1 - DOLPHIN D., POULSON R. et AVRAMOVIC O. Eds. (1989) Glutathione : chemical, biochemical and medical aspects, Vol. I&II, J.WILEY and Sons

2 - ANDERSON M.E. (1989) dans DOLPHIN D., POULSON R. et AVRAMOVIC O.Eds., Glutathione : chemical, biochemical and medical aspects, Vol. I&II, J.WILEY and Sons, pp. 339-365

3 - TIETZE F.(1969) Anal. Biochem., <u>27</u>, 502-522

4 - LING. B. LIN, DEWAELE C., BAEYENS W.R.G. (1991) J. Chromatogr., <u>553</u>(1-2), 433-9

5 - SOFIC E., RIEDERER P., BURGER R., GSELL W., HEUSCHNEIDER G. (1991) Fresenius. J. Anal. Chem., <u>339</u>, 258-260

6 - ELLMAN G.L. (1959) Arch. Biochem. Biophys., <u>82</u>, 70-77

7 - SEDLAK J. et LINDSAY R.H. (1968) Anal. Biochem., <u>25</u>, 192-205

8 - LUTHRA N.P., DUNLAP B. et ODOM J.D. (1981) Anal. Biochem., <u>117</u>, 94-102

9 - TORSHINSKI Y.M. (1981) Sulfur in Proteins, Pergamon Press, pp. 124-125

10 - FARISS M.W. et REED D.J. (1987) Meth. in Enzymol., <u>143</u>, 101-109

11 - REED D.J. et BEATTY P.W. (1980) Reviews in Biochemical Toxicology - Elsevier, <u>2</u>, 213-24

12 - KOSOWER E. M., KOSOWER N.S. et RADKOWSKY A. (1983), dans Functions of Gluthathione : biochemical, physiological, toxicological and clinical aspects, LARSSON A. et al. Eds., Raven press, New York, pp. 243-250

13 - RICE G.C., BUMP E. A., SHRIEVE D.C., LEE W. et KOVACS M. (1986) Cancer Res., <u>46</u>, 6105-5110

14 - HOLLOWAY C.J. (1987) J. of Chromatogr., <u>390</u>, 101-110

15 - MUTUS B., WAGNER J.D., TALPAS C.J., DIMMOCK J.R., PHILLIPS O.A., REID R.S. (1989) Anal. Biochem., <u>177</u>, 237-243

16 - GRIFFITH O. W. (1980) Anal. Biochem., <u>106</u>, 207-212

17 - HISSIN P., HILF R. (1976) Anal. Biochem., <u>74</u>, 214-226

18 - MARTENSSON J. (1987) J. of Chromatogr., <u>420</u>, 152-157

19 - FAHEY R.C. (1989), dans "Glutathione : chemical, biochemical and medical aspects", DOLPHIN D., POULSON R., AVRAMOVIC O. Eds., John Wiley and Sons, pp. 303-337

20 - BIN LING B., BAEYENS F.R.G., MARYSAEL H. (1989) Anal. Chim. Acta, <u>227</u>, 203-209

21 - INOUE M., SAITO Y., HIRATA E., MORINO Y., NAGASE S., (1987) J. Prot. Chem., <u>6</u>, 207-225

## Revendications

1. Procédé de dosage des mercaptans dans un échantillon aqueux, notamment biologique, caractérisé en ce qu'il comprend essentiellement la formation d'un produit de substitution chromophorique entre tous les mercaptans présents dans l'échantillon et un réactif choisi parmi les composés de type quinoléinium répondant à la formule générale I :

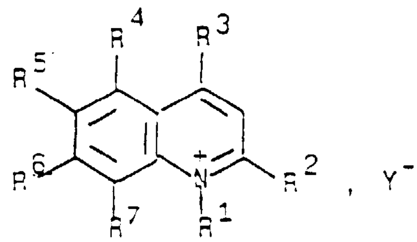

(I)

dans laquelle :

$R^1$ = alkyle (avec 1 à 6 atomes de carbone), benzyle, p-nitrobenzyle, phényle ou o,p-dinitrophényle;
$R^2$ = hydrogène ou méthyle;
$R^3$ = hydrogène, F, Cl, Br, I ou SCN;
$R^4$ = hydrogène, F, Cl, $NO_2$ ou $CF_3$;
$R^5$ = hydrogène, $NO_2$ ou Cl;
$R^6$ = hydrogène, F, Cl, $CF_3$ ou $NO_2$;
$R^7$ = hydrogène, OH, benzyloxy ou $NO_2$;

étant entendu que $R^3$, $R^4$, $R^5$ et $R^6$ ne peuvent représenter simultanément l'hydrogène; et
Y = fluorure, chlorure, bromure, iodure, méthylsulfate, fluorosulfonate, phosphate, tétrafluoroborate ou tosylate.

2. Procédé de dosage selon la revendication 1 du glutathion réduit (GSH) dans un échantillon aqueux, notamment biologique, caractérisé en ce qu'il comprend essentiellement les étapes consistant à :

   1. former un produit de substitution chromophorique entre tous les mercaptans présents dans l'échantillon et un réactif choisi parmi les composés de formule générale I définie à la revendication 1 ; et
   2. amener le pH du milieu réactionnel à une valeur comprise entre 12,8 et 13,8, pour former spécifiquement une thione chromophorique à partir du produit de substitution chromophorique formé à l'étape 1. entre le GSH et ledit réactif.

3. Procédé de dosage selon la revendication 1 des mercaptans totaux dans un échantillon aqueux, notamment biologique, caractérisé en ce qu'il comprend essentiellement les étapes consistant à :

   1. ajouter à l'échantillon à doser, tamponné dans la gamme de pH 7,0 à 7,5, un aliquot d'une solution-mère, dans l'eau ou dans un solvant miscible à l'eau, d'un réactif BXT de formule générale I en excès,
   2. homogénéiser le milieu réactionnel ainsi obtenu,
   3. laisser incuber pendant au moins 5 minutes,
   4. mesurer, à l'aide d'un spectrophotomètre, l'absorbance du ou des produit(s) de substitution ou adduit(s) formé(s) entre le réactif BXT et le ou les mercaptan(s) présent(s) dans l'échantillon et
   5. déterminer la concentration en moles/litre des mercaptans totaux présents dans l'échantillon, selon la formule :

$$[\text{mercaptans totaux}] = (A/\varepsilon m \times d) \times D$$

dans laquelle :

- A représente l'absorbance mesurée,

- εm représente le coefficient d'extinction molaire spécifique du réactif BXT utilisé, exprimé en $l.mole^{-1}.cm^{-1}$,
- d représente la longueur de la cuve, exprimée en cm, et
- D représente le coefficient de dilution.

**4.** Procédé de dosage selon la revendication 1 du glutathion réduit, GSH, dans un échantillon aqueux, notamment biologique, caractérisé en ce qu'il comprend essentiellement les étapes consistant à :

1. ajouter à l'échantillon à doser, tamponné dans la gamme de pH 7,0 à 7,5, un aliquot d'une solution-mère, dans l'eau ou dans un solvant miscible à l'eau, d'un réactif BXT de formule générale I en excès,
2. homogénéiser le milieu réactionnel ainsi obtenu,
3. laisser incuber pendant au moins 5 minutes,
4. ajuster le pH du mélange à une valeur comprise entre 12,8 et 13,8, selon le réactif BXT choisi,
5. laisser incuber pendant au moins 15 minutes,
6. mesurer, à l'aide d'un spectrophotomètre, l'absorbance de la thione chromophorique produite par β-élimination, spécifiquement avec l'adduit GSH-réactif BXT et
7. déterminer la concentration en moles/litre du GSH dans l'échantillon, selon la formule :

$$[GSH] = (A'/\varepsilon m \times d) \times D$$

dans laquelle :

- A' représente l'absorbance mesurée,
- εm représente le coefficient d'extinction molaire spécifique du réactif BXT utilisé, exprimé en $l.mole^{-1}.cm^{-1}$,
- d représente la longueur de la cuve, exprimée en cm, et
- D représente le coefficient de dilution.

**5.** Procédé de dosage selon la revendication 1 de la concentration des mercaptans distincts du glutathion dans un échantillon aqueux, notamment biologique, au moyen de la différence des concentrations mesurées respectivement selon les procédés des revendications 3 et 4, en utilisant la formule :

$$[\text{mercaptans distincts du GSH}] = [\text{mercaptans totaux}] - [GSH].$$

**6.** Procédé selon l'une quelconque des revendications 1 à 5, caractérisé en ce que le réactif utilisé est le réactif BXT03015 ou tétrafluoroborate de 4-chloro-7-trifluorométhyl-1-méthylquinoléinium.

**7.** Nécessaire ou kit pour la mise en oeuvre du procédé selon l'une quelconque des revendications 1 à 5, caractérisé en ce qu'il comprend essentiellement, en tant que réactif, un composé de formule générale I telle que définie à la revendication 1.

**8.** Nécessaire ou kit selon la revendication 7, caractérisé en ce que le composé de formule générale I précité est choisi parmi le groupe consistant du tétrafluoroborate de 4-chloro-7-trifluorométhyl-1-méthylquinoléinium, le tétrafluoroborate de 4-chloro-1,2-diméthylquinoléinium; le tétrafluoroborate de 4-chloro-1-méthylquinoléinium, le tétrafluoroborate de 7-chlore-1,2-diméthylquinoléinium, le tétrafluoroborate de 4,7-dichloro-1-méthylquinoléinium, le tétrafluoroborate de 5-chloro-8-hydroxy-1-méthylquinoléinium, le tétrafluoroborate de 5-chloro-8-benzyloxy-1-méthylquinoléinium, le tétrafluoroborate de 5-fluoro-1-méthylquinoléinium, le fluorosulfonate de 5-fluoro-8-nitro-1-méthylquinoléinium, le tétrafluoroborate de 5-nitro-1-méthylquinoléinium, le fluorosulfonate de 6-chloro-7-trifluorométhyl-1-méthylquinoléinium et le fluorosulfonate de 6-chloro-5-trifluorométhyl-1-méthylquinoléinium.

**9.** Nécessaire ou kit selon la revendication 7 ou 8, caractérisé en ce que le composé de formule générale I précité est le tétrafluoroborate de 4-chloro-7-trifluorométhyl-1-méthylquinoléinium.

## Patentansprüche

**1.** Verfahren zur Messung von Mercaptanen in einer wässerigen, insbesondere biologischen Probe, dadurch gekennzeichnet, daß es im wesentlichen die Bildung eines chromophoren Substitutionsprodukts zwischen allen in der

Probe vorliegenden Mercaptanen und einem Reagens umfaßt, ausgewählt aus den Verbindungen vom Chinolinium-Typ der allgemeinen Formel (I):

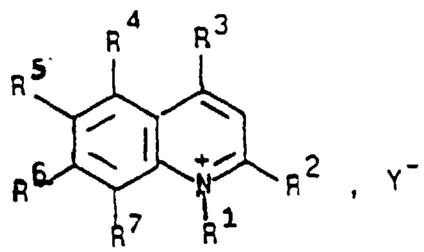

(I),

worin $R^1$ = Alkyl (mit 1 bis 6 Kohlenstoffatomen), Benzyl, p-Nitrobenzyl, Phenyl oder o,p-Dinitrophenyl; $R^2$ = Wasserstoff oder Methyl; $R^3$ = Wasserstoff, F, Cl, Br, I oder SCN; $R^4$ = Wasserstoff, F, Cl, $NO_2$ oder $CF_3$; $R^5$ = Wasserstoff, $NO_2$ oder Cl;
$R^6$ = Wasserstoff, F, Cl, $CF_3$ oder $NO_2$; $R^7$ = Wasserstoff, OH, Benzyloxy oder $NO_2$; mit der Maßgabe, daß $R^3$, $R^4$, $R^5$ und $R^6$ nicht gleichzeitig Wasserstoff bedeuten können; und Y = Fluorid, Chlorid, Bromid, Iodid, Methylsulfat, Fluorsulfonat, Phosphat, Tetrafluorborat oder Tosylat.

**2.** Verfahren nach Anspruch 1 zur Messung von reduziertem Glutathion (GSH) in einer wässerigen, insbesondere biologischen Probe, dadurch gekennzeichnet, daß es im wesentlichen die folgenden Schritte umfaßt:

1. Bilden eines chromophoren Substitutionsprodukts zwischen allen in der Probe vorliegenden Mercaptanen und einem Reagens, ausgewählt aus den Verbindungen der in Anspruch 1 definierten allgemeinen Formel (I); und
2. Führen des pH des Reaktionsmediums auf einen Wert zwischen 12,8 und 13,8, um spezifisch ein chromophores Thion aus dem in Schritt 1. gebildeten chromophoren Substitutionsprodukt zwischen dem GSH und dem Reagens zu bilden.

**3.** Verfahren nach Anspruch 1 zur Messung der gesamten Mercaptane in einer wässerigen, insbesondere biologischen Probe, dadurch gekennzeichnet, daß es im wesentlichen die folgenden Schritte umfaßt:

1. Zusetzen zur zu messenden Probe, die auf einen pH-Bereich von 7,0 bis 7,5 gepuffert wird, einer aliquoten Menge einer Mutterlauge, in Wasser oder in einem mit Wasser mischbaren Lösungsmittel, eines BXT-Reagens der allgemeinen Formel (I) im Überschuß;
2. Homogenisieren des so erhaltenen Reaktionsmediums;
3. Inkubierenlassen während zumindest 5 Minuten;
4. Messen, mit Hilfe eines Spektrometers, des Absorptionsvermögens des oder der Substitutionsprodukte oder des oder der Addukte, das bzw. der bzw. die zwischen dem BXT-Reagens und dem oder den Mercaptanen, die in der Probe vorliegen, gebildet werden;
5. Bestimmen der Konzentration in Mol/Liter der gesamten Mercaptane, die in der Probe vorliegen, gemäß der Formel:

$$[\text{gesamte Mercaptane}] = (A/\varepsilon m \times d) \times D,$$

worin A das gemessene Absorptionsvermögen bedeutet, εm den für das verwendete BXT-Reagens spezifischen molaren Extinktionskoeffizienten, ausgedrückt in $l.mol^{-1}.cm^{-1}$, darstellt, d die Länge der Küvette, ausgedrückt in cm, ist, und D den Verdünnungskoeffizienten bedeutet.

**4.** Verfahren nach Anspruch 1 zur Messung von reduziertem Glutathion (GSH) in einer wässerigen, insbesondere biologischen Probe, dadurch gekennzeichnet, daß es im wesentlichen die folgenden Schritte umfaßt:

1. Zusetzen zur zu messenden Probe, die auf einen pH-Bereich von 7,0 bis 7,5 gepuffert wird, einer aliquoten Menge einer Mutterlauge, in Wasser oder in einem mit Wasser mischbaren Lösungsmittel, eines BXT-Reagens der allgemeinen Formel (I) im Überschuß;

2. Homogenisieren des so erhaltenen Reaktionsmediums;
3. Inkubierenlassen während zumindest 5 Minuten;
4. Einstellen des pH der Mischung auf einen Wert zwischen 12,8 und 13,8, gemäß dem ausgewählten BXT-Reagens;
5. Inkubierenlassen während zumindest 15 Minuten;
6. Messen, mit Hilfe eines Spektrometers, des Absorptionsvermögens des durch β-Eliminierung erzeugten chromophoren Thions, spezifisch mit dem Addukt GSH-reaktives BTX; und
7. Bestimmen der Konzentration in Mol/Liter von GSH in der Probe, gemäß der Formel:

$$[GSH] = (A'/\varepsilon m \times d) \times D,$$

worin A' das gemessene Absorptionsvermögen bedeutet, εm den für das verwendete BXT-Reagens spezifischen molaren Extinktionskoeffizienten, ausgedrückt in $l.mol^{-1}.cm^{-1}$, darstellt, d die Länge der Küvette, ausgedrückt in cm, ist, und D den Verdünnungskoeffizienten bedeutet.

**5.** Verfahren nach Anspruch 1 zur Messung der Konzentration der von Glutathion verschiedenen Mercaptane in einer wässerigen, insbesondere biologischen Probe mit Hilfe der Differenz der jeweils gemäß den Verfahren der Ansprüche 3 und 4 gemessenen Konzentrationen unter Verwendung der Formel:

[von GSH verschiedene Mercaptane]=[gesamte Mercaptane]-[GSH].

**6.** Verfahren nach einem der Ansprüche 1 bis 5, dadurch gekennzeichnet, daß das verwendete Reagens das Reagens BXT03015 oder 4-Chlor-7-trifluormethyl-1-methylchinoliniumtetrafluorborat ist.

**7.** Testsatz oder Kit zur Durchführung des Verfahrens nach einem der Ansprüche 1 bis 5, dadurch gekennzeichnet, daß er im wesentlichen als Reagens eine Verbindung der allgemeinen Formel (I), wie in Anspruch 1 definiert, umfaßt.

**8.** Testsatz oder Kit nach Anspruch 7, dadurch gekennzeichnet, daß die Verbindung der Formel (I) ausgewählt ist aus der Gruppe bestehend aus 4-Chlor-7-trifluormethyl-1-methylchinoliniumtetrafluorborat, 4-Chlor-1,2-dimethylchinoliniumtetrafluorborat, 4-Chlor-1-methylchinoliniumtetrafluorborat, 7-Chlor-1,2-dimethylchinoliniumtetrafluorborat, 4,7-Dichlor-1-methylchinoliniumtetrafluorborat, 5-Chlor-8-hydroxy-1-methylchinoliniumtetrafluorborat, 5-Chlor-8-benzyloxy-1-methylchinoliniumtetrafluorborat, 5-Fluor-1-methylchinoliniumtetrafluorborat, 5-Fluor-8-nitro-1-methylchinoliniumfluorsulfonat, 5-Nitro-1-methylchinoliniumtetrafluorborat, 6-Chlor-7-trifluormethyl-1-methylchinoliniumfluorsulfonat und 6-Chlor-5-trifluormethyl-1-methylchinoliniumfluorsulfonat.

**9.** Testsatz oder Kit nach Anspruch 7 oder 8, dadurch gekennzeichnet, daß die Verbindung der allgemeinen Formel (I) 4-Chlor-7-trifluormethyl-1-methylchinoliniumtetrafluorborat ist.

### Claims

**1.** Process for assaying mercaptans in an aqueous sample, especially a biological sample, characterized in that it essentially comprises the formation of a chromophoric substitution product between all the mercaptans present in the sample and a reagent chosen from the quinolinium type compounds corresponding to general formula I:

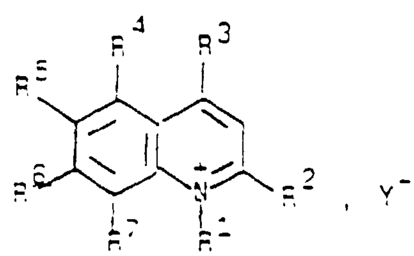

in which

$R^1$ = alkyl (with 1 to 6 carbon atoms), benzyl, p-nitrobenzyl, phenyl or o,p-dinitrophenyl;
$R^2$ = hydrogen or methyl;
$R^3$ = hydrogen, F, Cl, Br, I or SCN;
$R^4$ = hydrogen, F, Cl, $NO_2$ or $CF_3$;
$R^5$ = hydrogen, $NO_2$ or Cl;
$R^6$ = hydrogen, F, Cl, $CF_3$ or $NO_2$;
$R^7$ = hydrogen, OH, benzyloxy or $NO_2$;

it being understood that $R^3$, $R^4$, $R^5$ and $R^6$ cannot simultaneously represent hydrogen; and

Y = fluoride, chloride, bromide, iodide, methyl sulphate, fluorosulphonate, phosphate, tetrafluoroborate or tosylate.

**2.** Process for assaying according to claim 1 reduced glutathione (GSH) in an aqueous sample, especially a biological sample, characterized in that it essentially comprises the steps consisting of :

1. forming a chromophoric substitution product between all the mercaptans present in the sample and a reagent chosen from the compounds of general formula I defined in Claim 1; and
2. bringing the pH of the reaction medium to a value between 12.8 and 13.8, in order to form specifically a chromophoric thione from the chromophoric substitution product formed in step 1. between GSH and the said reagent.

**3.** Process for assaying according to claim 1 total mercaptans in an aqueous sample, especially a biological sample, characterized in that it comprises essentially the steps consisting of:

1. adding to the sample to be assayed, buffered within the pH range of 7.0 to 7.5, an aliquot of a stock solution, in water or a water-miscible solvent, of a BXT reagent of general formula I in excess,
2. homogenizing the reaction medium thus obtained,
3. leaving to incubate for at least 5 minutes,
4. measuring, by means of a spectrophotometer, the absorbance of the substitution product(s) or adduct(s) formed between the BXT reagent and the mercaptan(s) present in the sample and
5. determining the concentration, in moles/litre of total mercaptans present in the sample, according to the formula :

$$[\text{total mercaptans}] = (A/\varepsilon m \times d) \times D$$

in which:

- A represents the absorbance measured,
- εm represents the specific molar extinction coefficient for the BXT reagent used, expressed in $l.mole^{-1}.cm^{-1}$,
- d represents the cuvette length, expressed in cm, and
- D represents the coefficient of dilution.

**4.** Process for assaying according to claim 1 reduced glutathione, GSH, in an aqueous sample, especially a biological sample, characterized in that it comprises essentially the steps consisting of:

1. adding to the sample to be assayed, buffered within the pH range of 7.0 to 7.5, an aliquot of a stock solution, in water or in a water-miscible solvent, of a BXT reagent of general formula I in excess,
2. homogenizing the reaction medium thus obtained,
3. leaving to incubate for at least 5 minutes,
4. adjusting the pH of the mixture to a value of between 12.8 and 13.8, according to the BXT reagent chosen,
5. leaving to incubate for at least 15 minutes,
6. measuring, by means of a spectrophotometer, the absorbance of the chromophoric thione produced by β-elimination, specifically with the adduct GSH-BXT reagent and

7. determining the concentration in moles/litre of GSH in the sample, according to the formula:

$$[GSH] = (A'/\varepsilon m \times d) \times D$$

in which:

- A' represents the absorbance measured,
- εm represents the specific coefficient of molar extinction of the BXT reagent used, expressed in $l.mole^{-1}.cm^{-1}$,
- d represents the cuvette length, expressed in cm, and
- D represents the coefficient of dilution.

**5.** Process for assaying according to claim 1 the concentration of mercaptans distinct from glutathione in an aqueous sample, especially a biological sample, by subtracting the concentrations measured according to the processes of Claims 3 and 4 respectively, using the formula:

[mercaptans distinct from GSH] = [total mercaptans] - [GSH].

**6.** Process according to any one of Claims 1 to 5, characterized in that the reagent used is the reagent BXT03015 or 4-chloro-7-trifluoromethyl-1-methylquinolinium tetrafluoroborate.

**7.** Set or kit for the implementation of the process according to any one of Claims 1 to 5, characterized in that it comprises essentially, as reagent, a compound of general formula I as defined in Claim 1.

**8.** Set or kit according to Claim 7, characterized in that the aforesaid compound of general formula I is chosen from the group consisting of the 4-chloro-7-trifluoromethyl-1-methylquinolinium tetrafluoroborate, 4-chloro-1,2-dimethylquinolinium tetrafluoroborate; 4-chloro-1-methylquinolinium tetrafluoroborate, 7-chloro-1 ,2-dimethylquinolinium tetrafluoroborate, 4,7-dichloro-1-methylquinolinium tetrafluoroborate, 5-chloro-8-hydroxy-1-methylquinolinium tetrafluoroborate, 5-chloro-8-benzyloxy-1-methylquinolinium tetrafluoroborate, 5-fluoro-1-methylquinolinium tetrafluoroborate, 5-fluoro-8-nitro-1-methylquinolinium fluorosulfonate, 5-nitro-1-methylquinolinium tetrafluoroborate, 6-chloro-7-trifluoromethyl-1-methylquinolinium fluorosulfonate and 6-chloro-5-trifluoromethyl-1-methylquinolinium fluorosulfonate.

**9.** Set or kit according to Claim 7 or 8, characterized in that the aforesaid compound of general formula I is 4-chloro-7-trifluoromethyl-1-methylquinolinium tetrafluoroborate.

Fig. 1

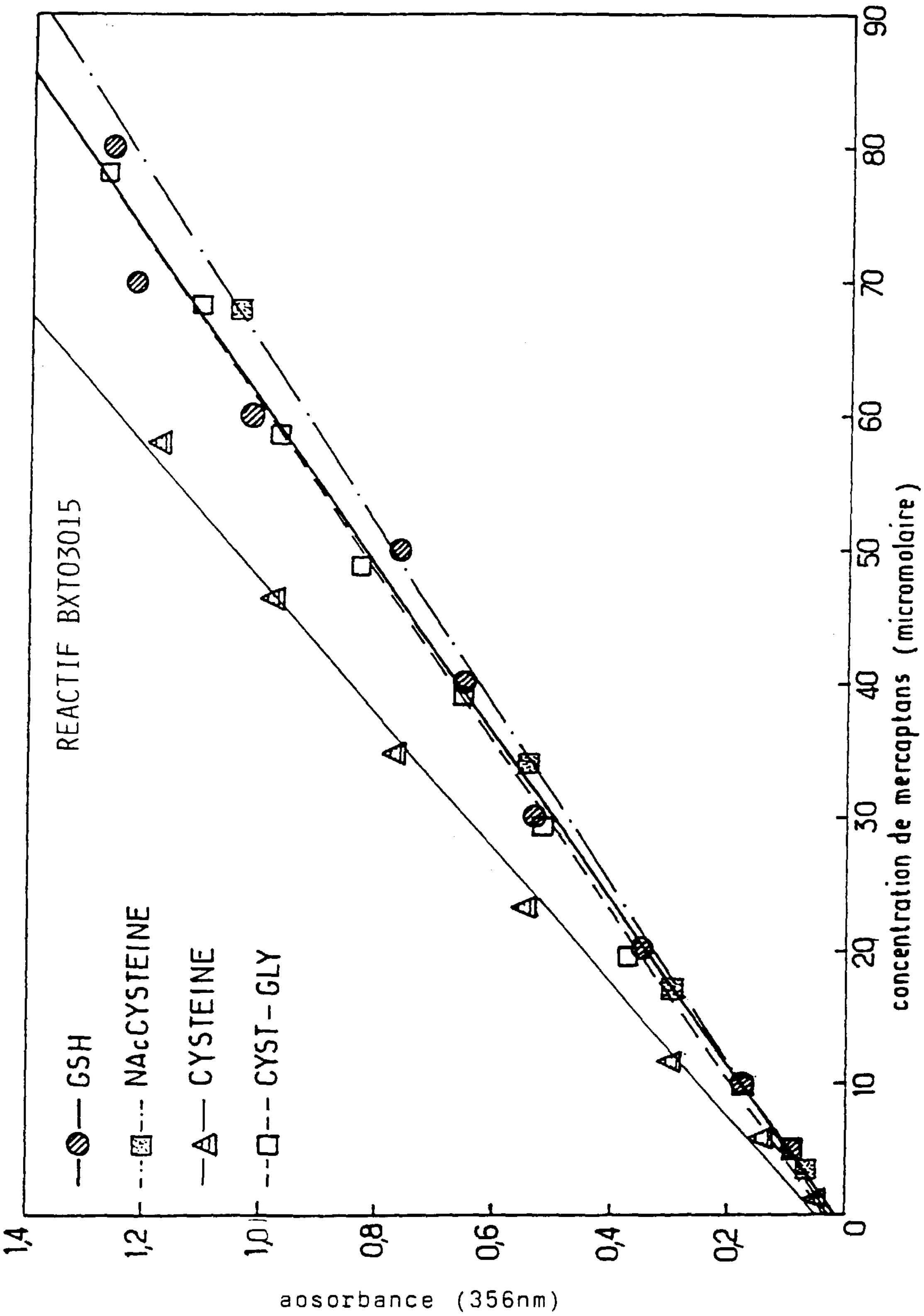

GSH
NAcCYSTEINE
CYSTEINE
CYST-GLY
REACTIF BXT03015
concentration de mercaptans (micromolaire)
0
10
20
30
40
50
60
70
80
90
absorbance (356nm)
0
0,2
0,4
0,6
0,8
1,0
1,2
1,4

Fig. 2

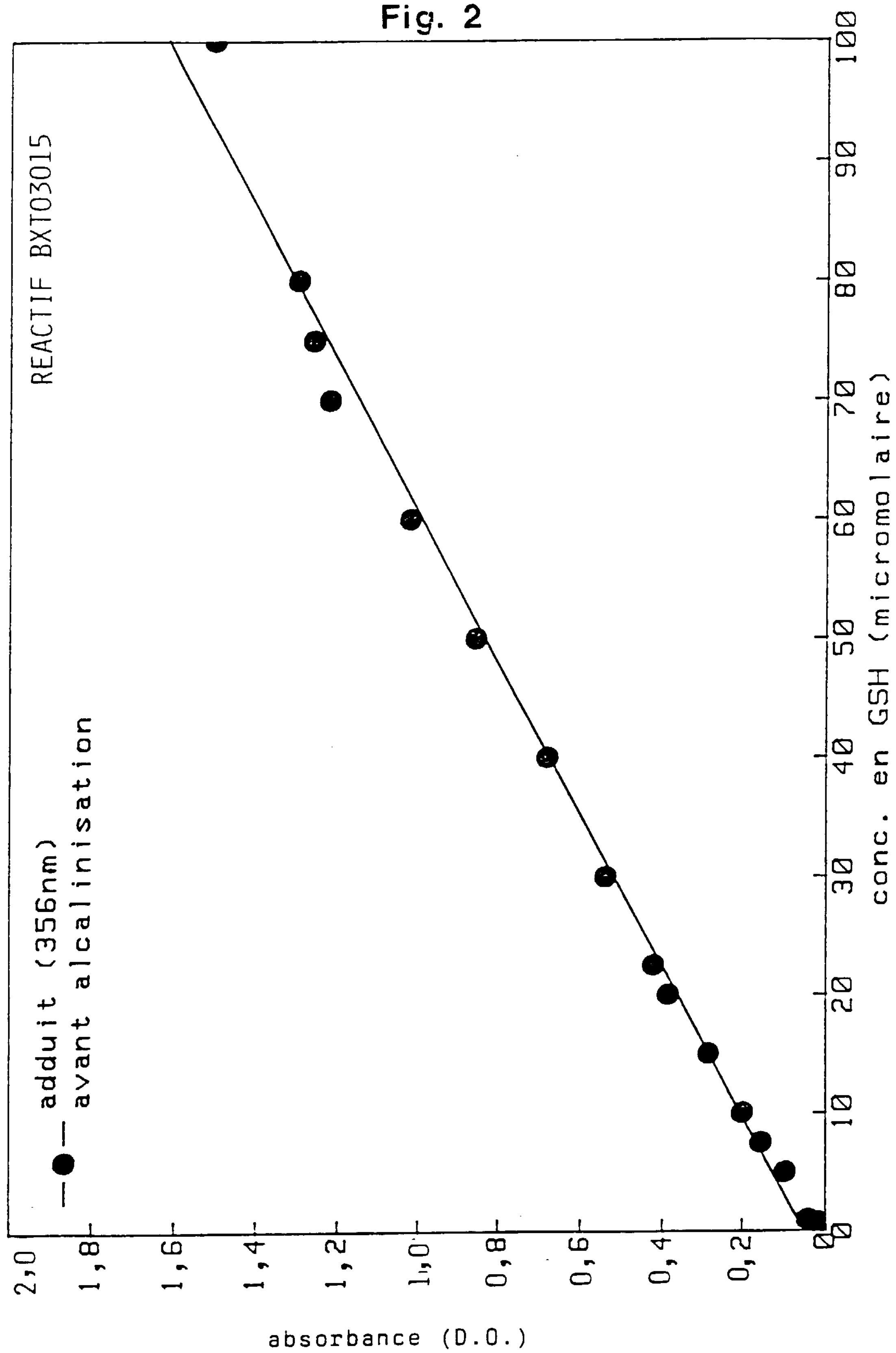
REACTIF BXTO3015
adduit (356nm)
avant alcalinisation
conc. en GSH (micromolaire)
absorbance (D.O.)
0
10
20
30
40
50
60
70
80
90
100
0,2
0,4
0,6
0,8
1,0
1,2
1,4
1,6
1,8
2,0

Fig. 3

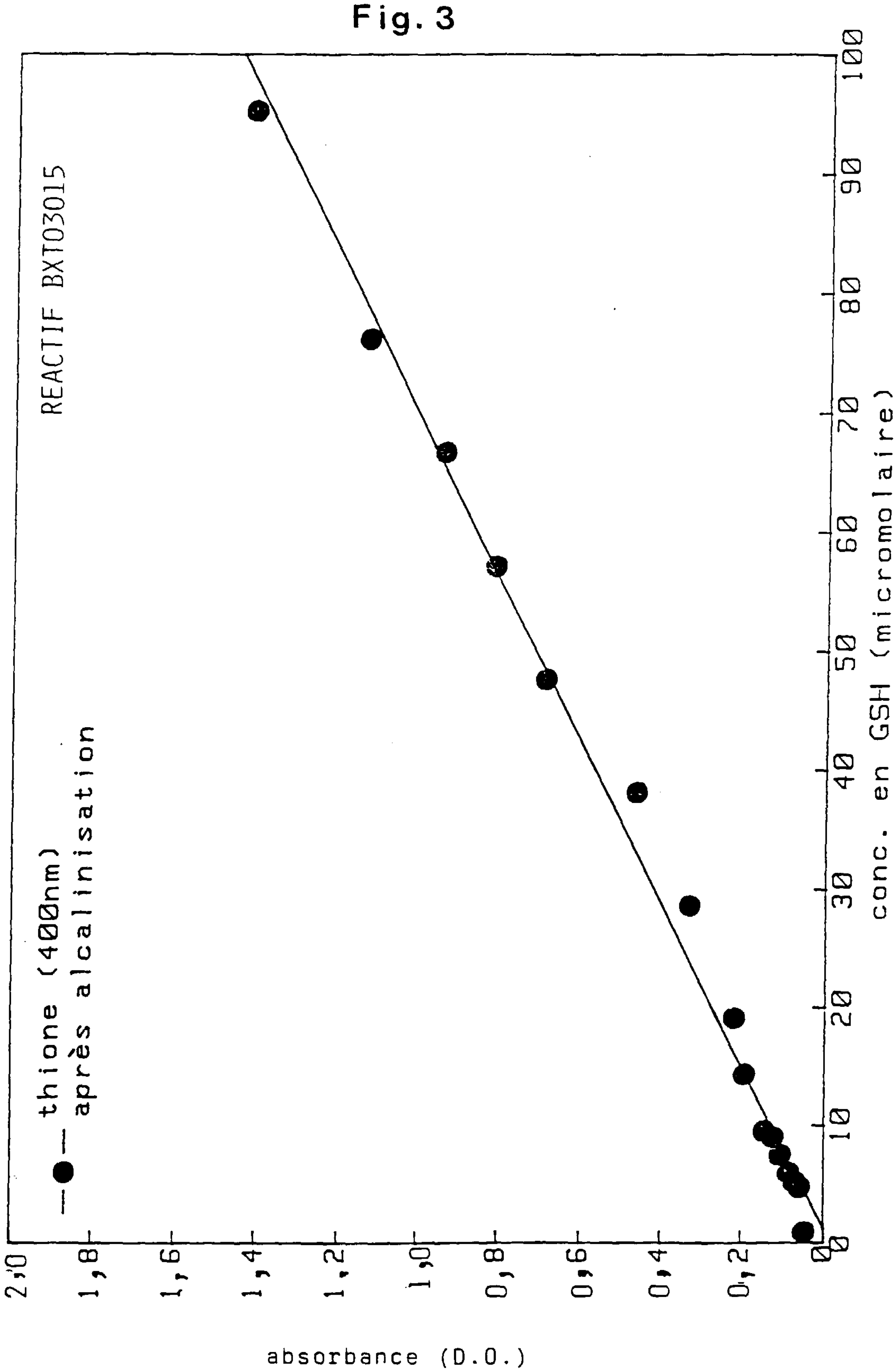
thione (400nm)
après alcalinisation
REACTIF BXT03015
conc. en GSH (micromolaire)
absorbance (D.O.)
0
10
20
30
40
50
60
70
80
90
100
0,2
0,4
0,6
0,8
1,0
1,2
1,4
1,6
1,8
2,0